# EUROPEAN PATENT APPLICATION

(11) **EP 4 813 293 A1**
(43) Date of publication of application: **30.09.2026**
(21) Application number: 23959117.5
(22) Date of filing: 21.11.2023
(51) Int. Cl.: A61B 5/145, A61B 5/1473, A61B 5/1486, A61B 5/151

(54) **APPLICATOR FOR INSERTING IN-VIVO ANALYTE SENSOR**

(71) Applicant: Shenzhen Savorcare Medical Technology Co., Ltd., Shenzhen, Guangdong 518102 (CN)
(72) Inventor: WANG, Xiaoqi, Shenzhen, Guangdong 518000 (CN); ZHU, Lianbing, Shenzhen, Guangdong 518000 (CN)
(74) Representative: Agca Kizil, Tugce
(86) International application number: PCT/CN2023/132853
(87) International publication number: WO 2025/107142

(57) **Abstract**

The present application discloses an applicator for inserting an in vivo analyte sensor. The applicator includes a base, a contact member, a transmitter bracket, a locking portion, a guide needle bracket, an elastic member, a transmitter and a guide needle. The contact member is movably connected to the base. The transmitter bracket is connected to the base. The locking portion is disposed on the transmitter bracket or the base. The locking portion is in contact with the guide needle bracket to lock the guide needle bracket on the transmitter bracket. The contact member is provided with an unlocking portion, and the base is configured to be selectively movable toward or away from a contact side of the contact member. There is no need for a user to perform assembly before use, which simplifies the structure of the applicator and the user's operation process. During use, a user presses the base while holding the contact member firmly against human skin to unlock the guide needle bracket, so that the guide needle can be driven to retract by the elastic member. The user does not need to perform additional operations for retracting the guide needle, making the retraction process of the guide needle more reliable.

## Description

### Technical Field

The present application relates to the technical field of medical devices, and in particular, to an applicator for inserting an in vivo analyte sensor.

### Background Art

With the improvement of people's living standards, the number of people with diabetes is also increasing day by day. Diabetes can lead to many complications such as coma, poisoning, neuropathy, and cardiovascular and cerebrovascular diseases. Therefore, diabetic patients need to know whether their glucose levels are within a safe range. However, traditional measurement methods are cumbersome, involve deep incisions, cause significant pain, and can only measure a single value, making it impossible for diabetic patients to know their glucose levels in real time. To enable diabetic patients to monitor their glucose levels in real time, some patients choose to wear analyte sensors. When wearing an analyte sensor, an injection device (applicator) is required. The analyte sensor is inserted into the human body via a sharp object (guide needle) of the injection device, so that the analyte sensor comes into contact with bodily fluids.

However, most existing injection devices include two components: a needle introducer and a transmitter. A user needs to assemble the needle introducer and the transmitter before use. This assembly process increases the difficulty of use for the user. Insufficient user training, complex pre-assembly steps and poor operating proficiency will easily cause unsuccessful insertion of the analyte sensor. Moreover, due to a lack of experience, it is difficult for the user to determine whether the sharp object of the injection device has been inserted properly, which may lead to the sharp object being retracted prematurely before the sensor is correctly implanted into the human body. Therefore, there is an urgent need for an applicator with a simpler structure and operation process, as well as more reliable retraction of the sharp object.

### Summary of the Invention

The main objective of the present application is to provide an applicator with a simpler structure and operation process, as well as more reliable retraction of the sharp object.

In an embodiment, an applicator for inserting an in vivo analyte sensor is provided, including:
a base;
a contact member, the contact member being movably connected to the base, wherein a side of the contact member facing a human body is defined as a contact side;
a transmitter bracket connected to the base;
a locking portion disposed on the transmitter bracket or the base;
a guide needle bracket disposed on the transmitter bracket, wherein the locking portion is in contact with the guide needle bracket to lock the guide needle bracket on the transmitter bracket;
an elastic member;
a transmitter connected to the transmitter bracket; and
a guide needle connected to the guide needle bracket;
wherein the contact member is provided with an unlocking portion; the base is configured to be movable toward the contact side to drive the unlocking portion into contact with the transmitter bracket, thereby releasing the locking of the guide needle bracket by the locking portion; the elastic member is configured to drive the guide needle bracket to move away from the transmitter bracket when the locking portion is released, thereby driving the guide needle to retract from the human body.

In an embodiment, the locking portion is snap-engaged with the guide needle bracket.

In an embodiment, the locking portion includes an elastic arm and a locking catch; the locking catch protrudes from the elastic arm and is snap-engaged with the guide needle bracket; when the unlocking portion comes into contact with the elastic arm, the elastic arm is driven to undergo elastic deformation, thereby releasing the snap engagement between the locking catch and the guide needle bracket.

In an embodiment, an end of the elastic arm is provided with a bent portion that bends toward an inner wall of the base, and the bent portion is configured to come into contact with the unlocking portion to release the snap engagement between the locking catch and the guide needle bracket

In an embodiment, the transmitter bracket further comprises a transmitter mounting portion, the transmitter is connected to the transmitter mounting portion, and the guide needle bracket is disposed on a side of the transmitter mounting portion facing the base; one end of the elastic arm is connected to the side of the transmitter mounting portion facing the base, and the other end of the elastic arm extends through the guide needle bracket; the locking catch is located on a side wall of the elastic arm facing the guide needle bracket, and the locking catch is snap-engaged with a top portion of the guide needle bracket; the bent portion is disposed at an end of the elastic arm facing away from the guide needle bracket.

In an embodiment, a plurality of locking portions are arranged around the center of the transmitter mounting portion; a plurality of unlocking portions are provided on the contact member, and the plurality of unlocking portions are arranged in one-to-one correspondence with the plurality of locking portions.

In an embodiment, when the unlocking portion comes into contact with the bent portion, the end of the elastic arm facing away from the transmitter mounting portion is driven to contract toward the center of the transmitter mounting portion, thereby buffering the movement of the guide needle bracket away from the transmitter mounting portion.

In an embodiment, the base is provided with an accommodating cavity, the accommodating cavity is provided with a first opening on a side facing the human body, and the contact member is movably disposed in the accommodating cavity; when the base moves toward or away from the contact side, the contact member is retracted into or extended out of the accommodating cavity from the first opening; when the base moves toward the contact side, the guide needle and part of the transmitter are driven to be inserted into the human body, and then the locking of the guide needle bracket by the locking portion is released.

In an embodiment, a first limiting groove is provided on an inner wall of the accommodating cavity, and the first limiting groove extends in a direction perpendicular to the first opening; the contact member comprises a contact body and a limiting portion, the limiting portion is connected to the contact body and movably disposed in the first limiting groove to guide the contact body to move along an extending direction of the first limiting groove.

In an embodiment, a resistance rib is provided on an inner wall of the accommodating cavity; the contact member comprises a contact body and a resistance arm, the contact body is movably connected to the inner wall of the accommodating cavity, one end of the resistance arm is connected to the contact body, and the other end of the resistance arm is provided with a resistance arm catch which is configured to cooperate with the resistance rib.

In an embodiment, a first positioning arm is provided on the inner wall of the accommodating cavity, and the first positioning arm is provided with a positioning groove; the transmitter bracket comprises a transmitter mounting portion and a second positioning arm, one end of the second positioning arm is connected to the transmitter mounting portion, the other end of the second positioning arm is provided with a positioning arm catch, and the positioning arm catch is snap-engaged with the positioning groove.

In an embodiment, the applicator further comprises a cover, wherein the cover is detachably connected to the base to close the first opening.

In an embodiment, the transmitter bracket comprises a transmitter mounting portion, a side of the transmitter mounting portion facing the first opening is provided with a mounting groove, the transmitter is mounted in the mounting groove, the guide needle extends out from a middle portion of the transmitter, and an analyte sensor of the transmitter is disposed in the guide needle.

In an embodiment, the guide needle bracket is disposed on a side of the transmitter mounting portion facing away from the first opening; the guide needle bracket is provided with a receiving cavity, and a second opening is formed on a side of the receiving cavity facing the transmitter mounting portion; the elastic member is disposed in the receiving cavity, one end of the elastic member abuts against an inner wall of the guide needle bracket, and the other end thereof abuts against the transmitter mounting portion.

In an embodiment, a middle portion of the guide needle bracket is provided with a connecting arm, the connecting arm is provided with a connecting catch, a rear end of the guide needle is provided with a connecting groove, and the connecting groove is snap-engaged with the connecting catch; the transmitter mounting portion is provided with a connecting through hole opposite to the connecting arm, and the guide needle passes through the transmitter mounting portion via the connecting through hole.

In an embodiment, a side of the guide needle bracket facing away from the second opening is provided with a plurality of first clearance holes; the locking portion comprises an elastic arm, and the first clearance holes are configured to allow the elastic arm to pass through.

In an embodiment, a second limiting groove is provided on an inner side wall of the guide needle bracket, and an extending direction of the second limiting groove is perpendicular to the second opening; the locking portion includes an elastic arm, and the second limiting groove is configured to cooperate with the elastic arm to guide the guide needle bracket to move in a direction perpendicular to the second opening.

In an embodiment, a mounting arm is provided on a side wall of the mounting groove, a mounting catch is provided on a side of the mounting arm facing the transmitter, the transmitter is provided with an engaging slot corresponding to the mounting catch, and the mounting catch is snap-engaged with the engaging slot.

In an embodiment, the contact member is provided with an inner cavity, and a side of the inner cavity facing the human body is provided with a third opening; the transmitter mounting portion and the guide needle bracket are located in the inner cavity; a side of the contact member facing away from the third opening is provided with a through hole, the guide needle bracket is arranged in the through hole, and the through hole is configured to allow the guide needle bracket to move away from the transmitter mounting portion.

In an embodiment, a plurality of second clearance holes are disposed on a side of the contact member away from the third opening; the base comprises a first positioning arm, and the second clearance holes are configured to allow the first positioning arm to pass through.

In an embodiment, an inner wall of the contact member is further provided with a limiting structure, and the limiting structure is configured to cooperate with the transmitter bracket and/or the guide needle bracket.

The applicator for inserting an in vivo analyte sensor according to the above embodiments includes a base, a contact member, a transmitter bracket, a locking portion, a guide needle bracket, an elastic member, a transmitter, and a guide needle. The contact member is movably connected to the base, and a side of the contact member facing a human body is defined as a contact side. The transmitter bracket is connected to the base, and the locking portion is disposed on the transmitter bracket or the base. The guide needle bracket is disposed on the transmitter bracket, and the locking portion is in contact with the guide needle bracket to lock the guide needle bracket on the transmitter bracket. The transmitter is connected to the transmitter bracket, and the guide needle is connected to the guide needle bracket. The contact member is provided with an unlocking portion; the base is configured to be movable toward the contact side to drive the unlocking portion into contact with the transmitter bracket, thereby releasing the locking of the guide needle bracket by the locking portion. The elastic member is configured to drive the guide needle bracket to move away from the transmitter bracket when the locking portion is released, thereby driving the guide needle to retract from the human body. On the one hand, the transmitter and the guide needle are pre-mounted on the transmitter bracket and the guide needle bracket, respectively, eliminating the need for a user to perform assembly before use, thereby simplifying the structure of the applicator and the user's operation process. On the other hand, during use, the user presses the base while holding the contact member firmly against human skin. When the base moves toward the contact side, the base drives the guide needle and part of the transmitter to be inserted into the human body, and drives the unlocking portion into contact with the transmitter bracket to unlock the guide needle bracket, thereby driving the guide needle to retract by an elastic restoring force of the elastic member. The entire operation process does not require the user to determine whether the guide needle is inserted in place, and no additional operations are required to retract the guide needle, reducing errors caused by subjective judgment and operation of the user, thereby making the retraction process of the guide needle more reliable.

### Brief Description of the Drawings

FIG. 1 is a schematic structural view showing an applicator in a closed state according to an embodiment of the present application;
FIG. 2 is a schematic structural view showing an applicator with a cover removed according to an embodiment of the present application;
FIG. 3 is a cross-sectional view showing an applicator in a closed state according to an embodiment of the present application;
FIG. 4 is a cross-sectional view showing an applicator with a cover removed according to an embodiment of the present application;
FIG. 5 is a cross-sectional view showing an applicator when a guide needle is in contact with the skin according to an embodiment of the present application;
FIG. 6 is a cross-sectional view showing an applicator when a guide needle is inserted into the skin according to an embodiment of the present application;
FIG. 7 is a schematic structural view showing a base according to an embodiment of the present application;
FIG. 8 is a schematic structural view showing a cover according to an embodiment of the present application;
FIG. 9 is a schematic structural view showing a transmitter bracket from one perspective according to an embodiment of the present application;
FIG. 10 is a schematic structural view showing a transmitter bracket from another perspective according to an embodiment of the present application;
FIG. 11 is a schematic structural view showing the interior of a guide needle bracket according to an embodiment of the present application;
FIG. 12 is a schematic structural view showing the back side of a guide needle bracket according to an embodiment of the present application;
FIG. 13 is a schematic structural view showing the exterior of a contact member according to an embodiment of the present application;
FIG. 14 is a schematic structural view showing the interior of a contact member according to an embodiment of the present application;
FIG. 15 is a schematic structural view showing a transmitter and a guide needle according to an embodiment of the present application;
FIG. 16 is a cross-sectional view showing an applicator with a cover removed according to another embodiment of the present application;

### Reference Numerals:

100, base; 110, accommodating cavity; 120, first opening; 130, first limiting groove; 140, protruding rib; 150, first positioning arm; 151, positioning groove; 160, base limiting rib; 170, resistance rib; 200, contact member; 210, contact side; 220, unlocking portion; 230, contact body; 240, resistance arm; 241, resistance arm catch; 250, inner cavity; 260, third opening; 270, through hole; 280, second clearance hole; 290, limiting structure; 291, columnar limiting rib; 292, limiting rib with an inclined surface; 2010, limiting portion; 300, transmitter bracket; 310, locking portion; 311, elastic arm; 312, locking catch; 313, bent portion; 320, transmitter mounting portion; 321, mounting groove; 322, mounting arm; 323, mounting catch; 324, slot; 325, connecting through hole; 330, second positioning arm; 331, positioning arm catch; 400, guide needle bracket; 410, receiving cavity; 420, second opening; 430, connecting arm; 431, connecting catch; 440, first clearance hole; 450, second limiting groove; 500, elastic member; 600, transmitter; 610, medical adhesive patch; 620, analyte sensor; 630, engaging slot; 700, guide needle; 710, connecting groove; 800, cover; 810, cover limiting surface.

### Detailed Description of Embodiments

The present application will be described in further detail below with reference to specific embodiments and accompanying drawings. Similar elements in different embodiments are denoted by corresponding similar reference numerals. In the following embodiments, numerous details are set forth to provide a better understanding of the present application. However, those skilled in the art will readily recognize that some of these features may be omitted under different circumstances, or may be replaced by other elements, materials, or methods. In some instances, certain operations associated with the present application are not shown or described in the specification, so as to prevent the core part of the present application from being obscured by excessive descriptions. For those skilled in the art, it is not necessary to describe these related operations in detail. They can fully understand the related operations based on the description in the specification and common general knowledge in the art.

In addition, the features, operations, or characteristics described in the specification may be combined in any suitable manner to form various embodiments. Meanwhile, the steps or actions in the method description can be rearranged or adjusted in a manner that is obvious to those skilled in the art. Therefore, the various sequences in the specification and drawings are only for the clear description of a particular embodiment, and are not intended to be mandatory unless a specific order is expressly required.

The serial numbers assigned to components herein, such as "first", "second", etc., are used to distinguish the described objects and have no sequential or technical meaning. The terms "connection" and "coupling" used in the present application, unless otherwise specified, include both direct and indirect connection (coupling).

In an embodiment, an applicator for inserting an in vivo analyte sensor is provided.

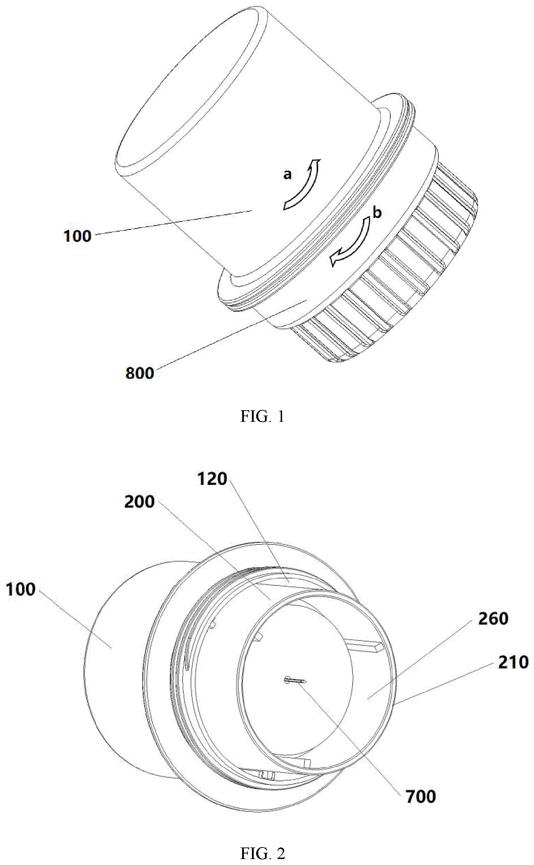

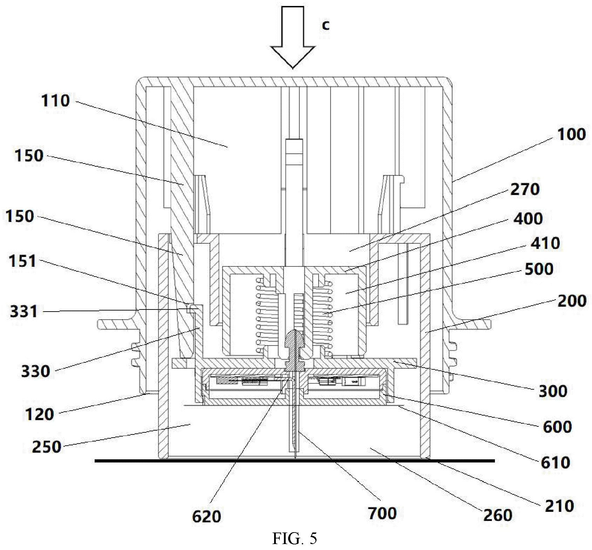

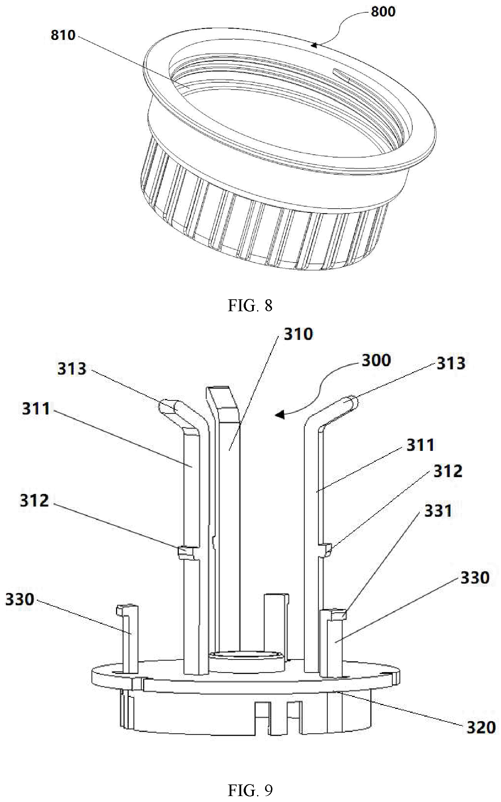

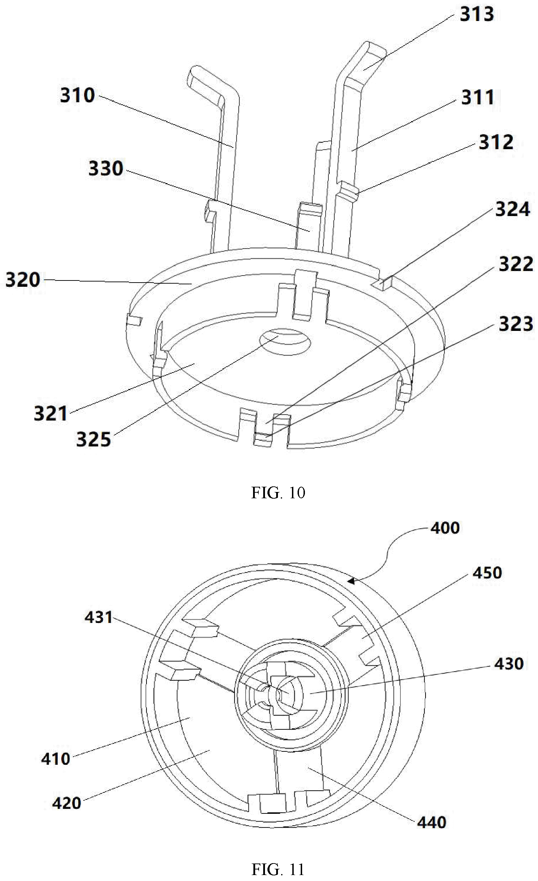

Referring to FIGS. 1-16, the applicator includes a base 100, a contact member 200, a transmitter bracket 300, a locking portion 310, a guide needle bracket 400, an elastic member 500, a transmitter 600, and a guide needle 700.

The contact member 200 is movably connected to the base 100, and a side of the contact member 200 facing a human body is defined as a contact side 210. The transmitter bracket 300 is connected to the base 100, and the locking portion 310 is disposed on the transmitter bracket 300 or the base 100. The guide needle bracket 400 is disposed on the transmitter bracket 300, and the locking portion 310 is in contact with the guide needle bracket 400 to lock the guide needle bracket 400 on the transmitter bracket 300. The elastic member 500 is disposed between the analyte sensor 620 and the guide needle bracket 400. The transmitter 600 is connected to the transmitter bracket 300, and the guide needle 700 is connected to the guide needle bracket 400. The contact member 200 is provided with an unlocking portion 220. The base 100 is configured to be selectively movable toward or away from the contact side 210 of the contact member 200. When the base 100 moves toward the contact side 210, the base 100 drives the guide needle 700 and part of the transmitter 600 to be inserted into the human body, and drives the unlocking portion 220 into contact with the transmitter bracket 300 to release the locking of the guide needle bracket 400 by the locking portion 310. The elastic member 500 is configured to drive the guide needle bracket 400 away from the transmitter bracket 300 when the locking portion 310 is released, thereby driving the guide needle 700 to retract from the human body.

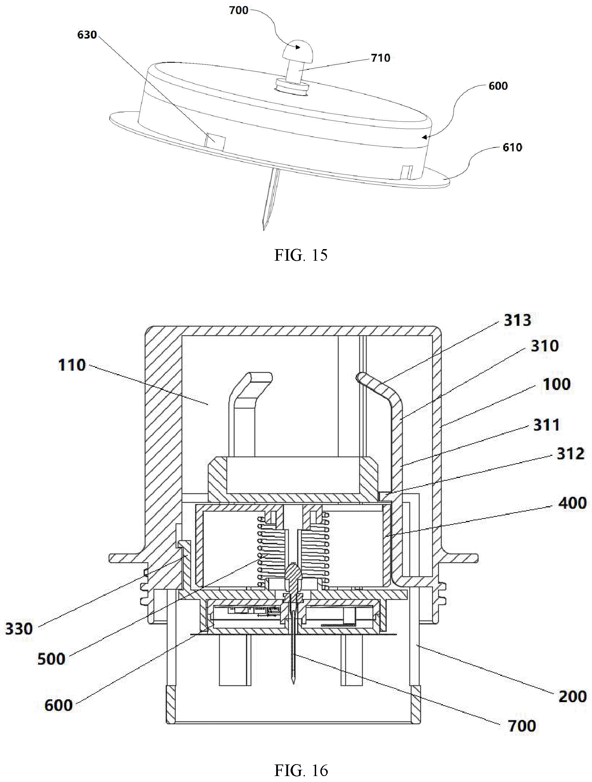

On the one hand, the transmitter 600 and the guide needle 700 are pre-mounted on the transmitter bracket 300 and the guide needle bracket 400, respectively, eliminating the need for a user to perform assembly before use, thereby simplifying the structure of the applicator and the user's operation process, and reducing the number of components to help reduce costs. On the other hand, during use, the user presses the base 100 while holding the contact member 200 firmly against human skin. When the base 100 moves toward the contact side 210, the base 100 drives the guide needle 700 and part of the transmitter 600 to be inserted into the human body, and drives the unlocking portion 220 into contact with the transmitter bracket 300 to unlock the guide needle bracket 400, thereby driving the guide needle 700 to retract by an elastic restoring force of the elastic member 500. The entire operation process does not require the user to determine whether the guide needle 700 is inserted in place, and no additional operations are required to retract the guide needle 700, reducing errors caused by subjective judgment and operation of the user, thereby making the retraction process of the guide needle 700 more reliable. Specifically, in an embodiment shown in FIGS. 1-15, the locking portion 310 is disposed on the transmitter bracket 300. In an embodiment shown in FIG. 16, the locking portion 310 is disposed on the base 100.

It should be noted that the transmitter 600 may be a transmitter that includes an electronic control unit or a transmitter that does not include an electronic control unit. The guide needle bracket 400 and the guide needle 700 may be directly connected, indirectly connected, or integrally formed via injection molding or other processes.

Referring to FIGS. 1 and 2, in an actual application scenario, a user first unscrews the cover 800 and removes the cover 800 from the base 100 to expose the contact member 200 and the guide needle 700. It should be noted that the tip of the guide needle 700 is spaced a certain distance from the edge of the third opening 260 at this time, and this distance ensures that the guide needle 700 does not pierce the skin immediately when the user attaches the contact member 200 to the human body. Specifically, the user may rotate the base 100 in a direction indicated by arrow a in FIG. 1 and rotate the cover 800 in a direction indicated by arrow b in FIG. 1, thereby unscrewing the cover 800.

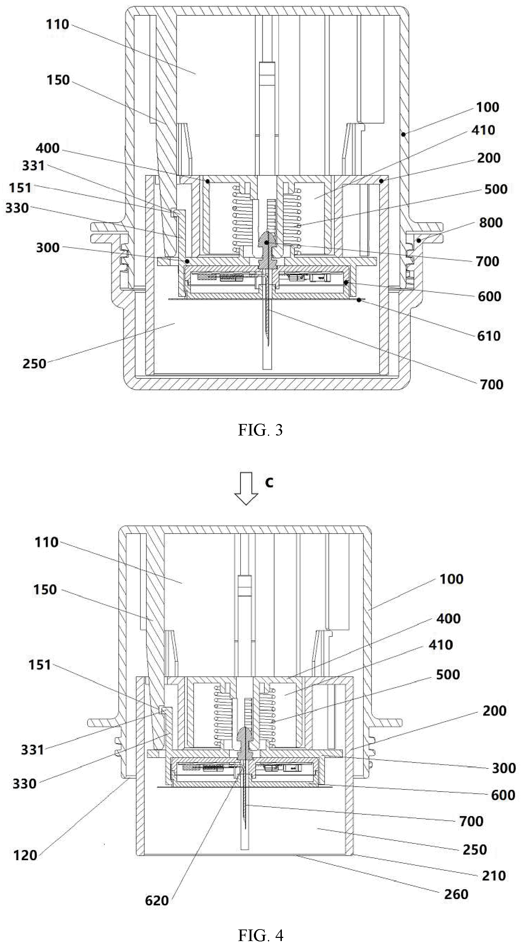

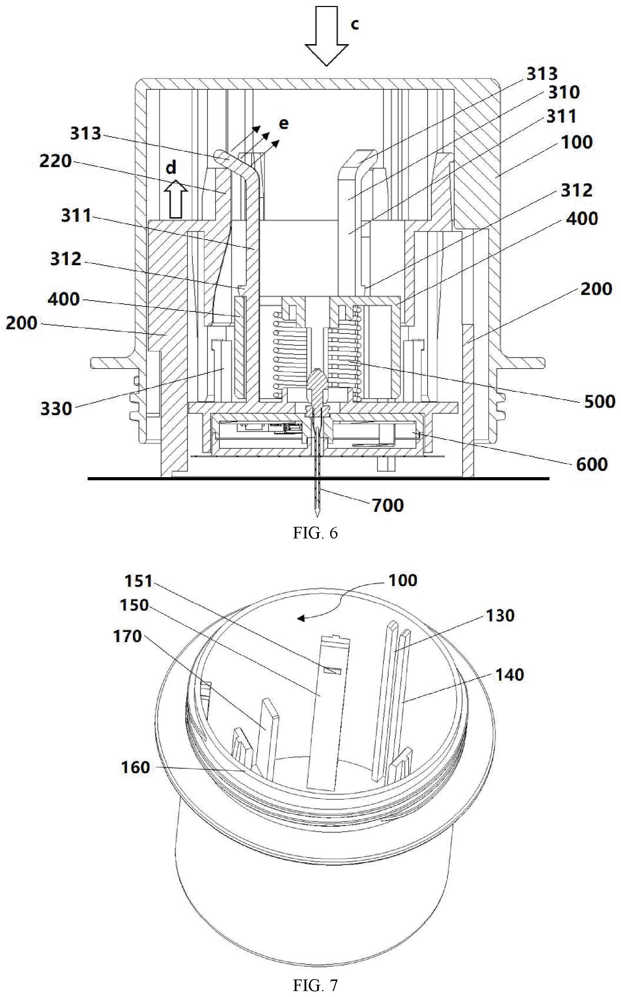

Next, referring to FIGS. 4-6, the user holds the base 100 with the cover 800 removed, brings the contact member 200 protruding from the base 100 into contact with a position on the human body where the sensor is to be worn, and then presses the base 100 toward the human body. Specifically, the user may press the base 100 in a direction indicated by arrow c in FIGS. 4-6. At this time, the contact member 200 remains stationary relative to the human body, and the base 100 and other components within the base 100 move toward the human skin. After moving a certain distance, the guide needle 700 begins to pierce the skin; after further moving a certain distance, the electrode wrapped by the guide needle 700 begins to enter the skin along the pierced wound. When the guide needle 700 is inserted into the human body to a sufficient depth, the unlocking portion 220 presses the bent portion 313 of the elastic arm 311, causing the elastic arm 311 to contract toward the center of the transmitter bracket 300, thereby releasing the locking catch 312 on the elastic arm 311 from the guide needle bracket 400. Then, the elastic member 500 pushes the guide needle bracket 400 away from the human body, and the guide needle bracket 400 pulls the guide needle 700 out of the human body, thereby achieving a needle retraction action. Specifically, the unlocking portion 220 moves relative to the base 100 in a direction indicated by arrow d in FIG. 6 and applies a force to the bent portion 313 in a direction indicated by arrow e.

Referring to FIGS. 1-6, in an embodiment, the locking portion 310 is snap-engaged with the guide needle bracket 400.

The locking portion 310 locks the guide needle bracket 400 via snap engagement. Specifically, the locking portion 310 and the guide needle bracket 400 may be snap-engaged via a catch, a slot, or other suitable snap structures.

Referring to FIGS. 1-6, in an embodiment, the locking portion 310 includes an elastic arm 311 and a locking catch 312. The locking catch 312 protrudes from the elastic arm 311 and is snap-engaged with the guide needle bracket 400. When the unlocking portion 220 comes into contact with the elastic arm 311, the elastic arm 311 is driven to undergo elastic deformation, thereby releasing the snap engagement between the locking catch 312 and the guide needle bracket 400.

The guide needle bracket 400 is locked by the snap engagement between the locking catch 312 and the guide needle bracket 400, so that the guide needle bracket 400 will not be pushed off the transmitter bracket 300 by the elastic member 500 in a locked state. When unlocking is required, the unlocking portion 220 presses the elastic arm 311, causing the elastic arm 311 to undergo elastic deformation, thereby driving the locking catch 312 to release the snap engagement with the guide needle bracket 400. Of course, in other embodiments, the locking manner between the locking portion 310 and the guide needle bracket 400 is not limited to snap engagement. For example, the locking portion 310 may also lock the guide needle bracket 400 by adhesive bonding, damping fit, magnetic fit, or other suitable manners.

Referring to FIGS. 6, 9 and 10, in an embodiment, an end portion of the elastic arm 311 is provided with a bent portion 313 that bends toward an inner wall of the base 100. The bent portion 313 is configured to come into contact with the unlocking portion 220 to release the snap engagement between the locking catch 312 and the guide needle bracket 400.

On the one hand, the bending causes the end portion of the elastic arm 311 to extend into a movement path of the unlocking portion 220, so that when the base 100 and the contact member 200 move relative to each other, the unlocking portion 220 can come into contact with the bent portion 313 of the elastic arm 311. On the other hand, an inclined surface where the bent portion 313 contacts the unlocking portion 220 serves as a guide, and can guide the unlocking portion 220 to gradually press the end portion of the elastic arm 311 toward the center of the transmitter bracket 300.

Referring to FIGS. 4-6, 9 and 10, in an embodiment, the transmitter bracket 300 further includes a transmitter mounting portion 320. The transmitter 600 is connected to the transmitter mounting portion 320, and the guide needle bracket 400 is disposed on a side of the transmitter mounting portion 320 facing the base 100. One end of the elastic arm 311 is connected to the side of the transmitter mounting portion 320 facing the base 100, and the other end extends through the guide needle bracket 400. The locking catch 312 is located on a side wall of the elastic arm 311 facing the guide needle bracket 400, and the locking catch 312 is snap-engaged with a top portion of the guide needle bracket 400. The bent portion 313 is disposed at an end of the elastic arm 311 facing away from the guide needle bracket 400.

On the one hand, the transmitter 600 is supported by the transmitter mounting portion 320. On the other hand, since the bent portion 313 is located at the end of the elastic arm 311 facing away from the guide needle bracket 400, when the user pushes the base 100 toward the human body, the unlocking portion 220 will not press the elastic arm 311 before coming into contact with the bent portion 313, so that the guide needle 700 can be inserted into the human body to a sufficient depth before triggering the needle retraction action.

Referring to FIGS. 4-6, 9 and 13, in an embodiment, a plurality of locking portions 310 are arranged around a center line of the transmitter mounting portion 320, a plurality of unlocking portions 220 are provided on the contact member 200, and the plurality of unlocking portions 220 are arranged in one-to-one correspondence with the plurality of locking portions.

On the one hand, the provision of the plurality of locking portions 310 helps to lock the guide needle bracket 400 more stably from a plurality of positions, thereby improving the safety of the applicator. On the other hand, since the plurality of unlocking portions 220 corresponding to the locking portions 310 are provided, when the user pushes the base 100 toward the human body, the plurality of unlocking portions 220 can come into contact with the plurality of locking portions 310 correspondingly, thereby enabling the multiple locking portions 310 to be unlocked at the same time, which helps to ensure the convenience of the unlocking process.

Referring to FIGS. 4-6, 9 and 10, in an embodiment, when the unlocking portion 220 comes into contact with the bent portion 313, an end of the elastic arm 311 facing away from the transmitter mounting portion 320 is driven to contract toward the center of the transmitter mounting portion 320, thereby buffering the movement of the guide needle bracket 400 away from the transmitter mounting portion.

During the needle retraction action, the guide needle bracket 400 and the guide needle 700 are pushed away from the transmitter bracket 300 by the elastic member 500. During this process, the elastic potential energy of the elastic member 500 is converted into the kinetic energy of the guide needle bracket 400 and the guide needle 700. If the guide needle bracket 400 directly impacts the base 100 at this time, severe impact and loud noise will be generated. In the present embodiment, when the unlocking portion 220 presses the end portion of the elastic arm 311, causing the end portion of the elastic arm 311 to contract toward the center of the transmitter mounting portion 320, the end portion of the elastic arm 311 extends into a movement path of the guide needle bracket 400 away from the transmitter mounting portion 320. This allows the guide needle bracket 400 to first come into contact with the elastic arm 311, and the elastic arm 311 acts as a buffer, thereby reducing impact and noise and improving the user experience.

Referring to FIGS. 3 and 7, in an embodiment, the base 100 is provided with an accommodating cavity 110. The accommodating cavity 110 is provided with a first opening 120 on a side facing the human body, and the contact member 200 is movably disposed in the accommodating cavity 110. When the base 100 moves toward or away from the contact side 210, the contact member 200 is retracted into or extended out of the accommodating cavity 110 from the first opening 120. When the base 100 moves toward the contact side 210, the guide needle 700 and part of the transmitter 600 are drived to be inserted into the human body, and then the locking of the guide needle bracket 400 by the locking portion 310 is released.

Part of the contact member 200 is accommodated in the accommodating cavity 110 of the base 100. When the user brings the contact member 200 into contact with the human body, part of the contact member 200 extends out of the accommodating cavity 110 through the first opening 120. When the user pushes the base 100 toward the human body, the part of the contact member 200 extending out of the accommodating cavity 110 is gradually retracted into the accommodating cavity 110 through the first opening 120.

Referring to FIGS. 3, 7 and 13, in an embodiment, a first limiting groove 130 is provided on an inner wall of the accommodating cavity 110, and the first limiting groove extends in a direction perpendicular to the first opening 120. The contact member 200 includes a contact body 230 and a limiting portion 2010. The limiting portion 2010 is connected to the contact body 230, and the limiting portion 2010 is movably disposed in the first limiting groove to guide the contact body 230 to move along an extension direction of the first limiting groove.

On the one hand, when a user pushes the base 100 toward the human body, the contact body 230 is guided to move along the extension direction of the first limiting groove 130 through the cooperation between the first limiting groove 130 and the limiting portion 2010, so that the contact member 200 can be retracted into the accommodating cavity 110 more smoothly. On the other hand, the cooperation between the first limiting groove 130 and the limiting portion 2010 can also prevent relative rotation between the contact member 200 and the base 100. Specifically, in an embodiment, the first limiting groove 130 is formed by two adjacent protruding ribs 140. In other embodiments, the first limiting groove 130 may also be formed by a recess in the inner wall of the accommodating cavity 110.

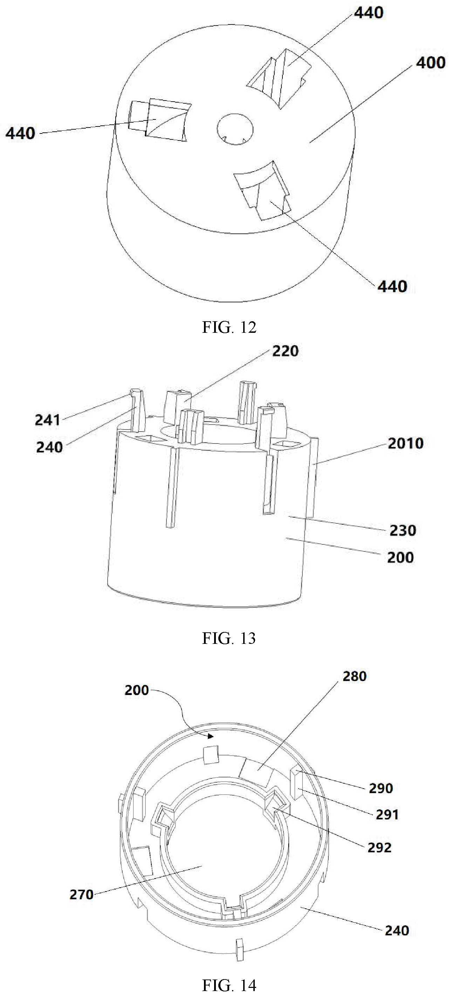

Referring to FIGS. 7 and 13, in an embodiment, a resistance rib 170 is provided on an inner wall of the accommodating cavity 110. The contact member 200 includes a contact body 230 and a resistance arm 240. The contact body 230 is movably connected to the inner wall of the accommodating cavity 110. One end of the resistance arm 240 is connected to the contact body 230, and the other end is provided with a resistance arm catch 241. The resistance arm catch 241 is configured to cooperate with the resistance rib 170.

The resistance arm catch 241 and the resistance rib 170 are combined to enhance the resistance when the user pushes the base 100, thereby improving the feedback when the user pushes the base 100 and reducing the possibility of false triggering of the guide needle 700.

Referring to FIGS. 3-5 and 9, in an embodiment, a first positioning arm 150 is provided on an inner wall of the accommodating cavity 110, and the first positioning arm 150 is provided with a positioning groove 151. The transmitter bracket 300 includes a transmitter mounting portion 320 and a second positioning arm 330. One end of the second positioning arm 330 is connected to the transmitter mounting portion 320, and the other end is provided with a positioning arm catch 331, which is snap-engaged with the positioning groove 151.

The snap engagement between the positioning arm catch 331 and the positioning groove 151 realizes the connection between the transmitter bracket 300 and the base 100, so that the transmitter bracket 300 can be driven to move when the user pushes the base 100. Of course, in other embodiments, the connection between the transmitter bracket 300 and the base 100 is not limited to snap engagement. For example, the transmitter bracket 300 may also be connected by adhesive bonding, screw connection or other suitable connection means.

Referring to FIGS. 1, 3 and 8, in an embodiment, the applicator further includes a cover 800, which is detachably connected to the base 100 to close the first opening 120.

When the applicator is not in use, the first opening 120 is closed by the cover 800 to protect the contact member 200 and the guide needle 700, and also prevent false triggering of the guide needle 700. Specifically, in an embodiment, an inner wall of the cover 800 is in threaded connection with an outer wall of the base 100. In other embodiments, the cover 800 and the base 100 may also be connected by snap connection, adhesive bonding, interference fit or the like. Specifically, a side of the applicator facing the cover 800 is provided with a base limiting rib 160, and the cover 800 is provided with a cover limiting surface 810 inside. The cover limiting surface 810 is configured to abut against the base limiting rib 160 to limit the assembly depth of the cover 800 relative to the base 100.

Referring to FIGS. 3, 9 and 10, in an embodiment, the transmitter bracket 300 includes a transmitter mounting portion 320. A side of the transmitter mounting portion 320 facing the first opening 120 is provided with a mounting groove 321. The transmitter 600 is mounted in the mounting groove 321. The guide needle 700 extends out from a middle portion of the transmitter 600, and an analyte sensor 620 of the transmitter 600 is disposed in the guide needle 700.

On the one hand, the transmitter 600 is mounted on the transmitter bracket 300 via the mounting groove 321, so that the transmitter 600 can be driven to move when the transmitter bracket 300 moves. On the other hand, since the analyte sensor 620 of the transmitter 600 is wrapped in the guide needle 700, the analyte sensor 620 can be driven into the human body when the guide needle 700 is inserted into the human body.

Referring to FIGS. 3, 9 and 10, in an embodiment, a mounting arm 322 is provided on a side wall of the mounting groove 321. A mounting catch 323 is provided on a side of the mounting arm 322 facing the transmitter 600. The transmitter 600 is provided with an engaging slot 630 corresponding to the mounting catch 323. The mounting catch 323 is snap-engaged with the engaging slot 630.

The transmitter 600 is fixed in the mounting groove 321 by the cooperation between the mounting catch 323 and the engaging slot 630. Specifically, a plurality of mounting catches 323 and a plurality of engaging slots 630 may be arranged in one-to-one correspondence around the circumference of the analyte sensor 620 to fix the transmitter 600 more stably. In other embodiments, the transmitter 600 may also be fixed in the mounting groove 321 by adhesive bonding or other suitable connection means.

Referring to FIGS. 3, 9 and 10, in an embodiment, an edge of the transmitter mounting portion 320 is provided with a slot 324. The slot 324 is configured to be in positioning cooperation with an inner wall of the contact member 200 to limit the direction of movement of the transmitter mounting portion 320 in the contact member 200.

Referring to FIGS. 3 and 15, in an embodiment, a side of the transmitter 600 configured to face the human body is provided with a medical adhesive patch 610, which allows the transmitter 600 to fit tightly against the human skin.

Referring to FIGS. 4-6, 11 and 12, in an embodiment, the guide needle bracket 400 is disposed on a side of the transmitter mounting portion 320 facing away from the first opening 120. The guide needle bracket 400 is provided with a receiving cavity 410, and a second opening 420 is formed on a side of the receiving cavity 410 facing the transmitter mounting portion 320. The elastic member 500 is disposed in the receiving cavity 410. One end of the elastic member 500 abuts against an inner wall of the guide needle bracket 400, and the other end abuts against the transmitter mounting portion 320.

The guide needle bracket 400 accommodates the elastic member 500 in a compressed state and part of the guide needle 700 through the receiving cavity 410. This allows the elastic member 500 in the receiving cavity 410 to recover deformation under its own elastic restoring force when the locking portion 310 releases the lock on the guide needle bracket 400, and pushes the guide needle bracket 400 to move away from the transmitter mounting portion 320. Specifically, in an embodiment, the elastic member 500 is configured as a spring. In other embodiments, the elastic member 500 may also be an elastic sheet or other suitable elastic structures.

Referring to FIGS. 4-6, 11 and 12, in an embodiment, a side of the guide needle bracket 400 facing away from the second opening 420 is provided with a plurality of first clearance holes 440. The locking portion 310 includes an elastic arm 311, and the first clearance holes 440 are configured to allow the elastic arm 311 to pass through.

Since the guide needle bracket 400 is provided with a first clearance hole 440, the elastic arm 311 of the transmitter bracket 300 can pass through the first clearance hole 440 and out of the receiving cavity 410, thereby preventing the receiving cavity 410 from interfering with the contact between the elastic arm 311 and the unlocking portion 220. Specifically, a plurality of first clearance holes 440 and a plurality of elastic arms 311 may be arranged in one-to-one correspondence along the circumference of the guide needle bracket 400.

Referring to FIGS. 4-6, 11 and 12, in an embodiment, a second limiting groove 450 is provided on an inner side wall of the guide needle bracket 400, and an extension direction of the second limiting groove 450 is perpendicular to the second opening 420. The base 100 includes a first positioning arm 150, the locking portion 310 includes an elastic arm 311, and the second limiting groove 450 is configured to cooperate with the elastic arm 311 to guide the guide needle bracket 400 to move in a direction perpendicular to the second opening 420.

When the elastic member 500 drives the guide needle bracket 400 to move away from the transmitter mounting portion 320, the guide needle bracket 400 is guided to move along the extension direction of the second limiting groove 450 through the cooperation between the second limiting groove 450 and the elastic arm 311, thereby helping to improve the stability of the direction of movement of the guide needle bracket 400.

Referring to FIGS. 4-6,11 and 12, in an embodiment, a middle portion of the guide needle bracket 400 is provided with a connecting arm 430, the connecting arm 430 is provided with a connecting catch 431. A rear end of the guide needle 700 is provided with a connecting groove 710, and the connecting groove 710 is snap-engaged with the connecting catch 431. The transmitter mounting portion 320 is provided with a connecting through hole 325 opposite to the connecting arm 430, and the guide needle 700 passes through the transmitter mounting portion 320 via the connecting through hole 325.

On the one hand, the guide needle 700 is fixed to the guide needle bracket 400 by the snap-fit cooperation between the connecting catch 431 and the connecting groove 710. On the other hand, the guide needle 700 passes through the transmitter mounting portion 320 via the connecting through hole 325, so that the transmitter mounting portion 320 does not obstruct the insertion of the guide needle 700 into the human body. Specifically, an end of the guide needle 700 that is inserted into the human body is a tip, and an end opposite to the tip is a rear end. In other embodiments, the connection between the guide needle 700 and the guide needle bracket 400 may also be adhesive bonding, interference fit or other suitable connection means.

Referring to FIGS. 4-6, 13 and 14, in an embodiment, the contact member 200 is provided with an inner cavity 250, and a side of the inner cavity 250 facing the human body is provided with a third opening 260. The transmitter mounting portion 320 and the guide needle bracket 400 are located in the inner cavity 250. A side of the contact member 200 facing away from the third opening 260 is provided with a through hole 270. The guide needle bracket 400 is located in the through hole 270. The through hole 270 is configured to allow the guide needle bracket 400 to move away from the transmitter mounting portion 320.

The inner cavity 250 of the contact member 200 accommodates the transmitter mounting portion 320 and the guide needle bracket 400. On the one hand, since the inner cavity 250 of the contact member 200 has the third opening 260, the base 100 can drive the guide needle 700 to pierce the human skin through the third opening 260. On the other hand, since the contact member 200 has the through hole 270, after the locking portion 310 releases the lock on the guide needle bracket 400, the guide needle bracket 400 can move out of the inner cavity 250 of the contact member 200 from the through hole 270, so that the contact member 200 does not obstruct the needle retraction action of the guide needle bracket 400.

Referring to FIGS. 4-6, 13 and 14, in an embodiment, a plurality of second clearance holes 280 are provided on a side of the contact member 200 facing away from the third opening 260. The base 100 includes a first positioning arm 150, and the second clearance holes 280 are configured to allow the first positioning arm 150 to pass through.

Since the contact member 200 is provided with the second clearance holes 280, the first positioning arm 150 of the base 100 can extend into the inner cavity 250 of the contact member 200 through the second clearance holes 280, so that the second positioning arm 330 on the transmitter bracket 300 in the inner cavity 250 can be snap-engaged with the first positioning arm 150. Specifically, a plurality of second clearance holes 280 and a plurality of first positioning arms 150 may be provided in one-to-one correspondence along the circumference of the contact member 200.

Referring to FIGS. 4-6, 13 and 14, in an embodiment, an inner wall of the contact member 200 is further provided with a limiting structure 290, and the limiting structure 290 is configured to cooperate with the transmitter bracket 300 and/or the guide needle bracket 400.

The limiting structure 290 limits the movement of the transmitter bracket 300 and/or the guide needle bracket 400 in the inner cavity 250. Specifically, referring to FIG. 14, the limiting structure 290 may include a columnar limiting rib 291 and a limiting rib 292 with an inclined surface. In other embodiments, the limiting structure 290 may also be configured in an arc shape, a triangular shape or other suitable shapes as needed.

The above uses specific embodiments to illustrate the present application, which are only used to facilitate understanding of the present application and are not intended to limit the present application. For those skilled in the art to which the present application pertains, several simple deductions, modifications or substitutions can be made based on the idea of the present application.

## Claims

1. An applicator for inserting an in vivo analyte sensor, comprising:
a base;
a contact member, the contact member being movably connected to the base, wherein a side of the contact member facing a human body is defined as a contact side;
a transmitter bracket connected to the base;
a locking portion disposed on the transmitter bracket or the base;
a guide needle bracket disposed on the transmitter bracket, wherein the locking portion is in contact with the guide needle bracket to lock the guide needle bracket on the transmitter bracket;
an elastic member;
a transmitter connected to the transmitter bracket; and
a guide needle connected to the guide needle bracket;
wherein the contact member is provided with an unlocking portion; the base is configured to be movable toward the contact side to drive the unlocking portion into contact with the transmitter bracket, thereby releasing the locking of the guide needle bracket by the locking portion; the elastic member is configured to drive the guide needle bracket to move away from the transmitter bracket when the locking portion is released, thereby driving the guide needle to retract from the human body.

2. The applicator according to claim 1, wherein the locking portion is snap-engaged with the guide needle bracket.

3. The applicator according to claim 2, wherein the locking portion includes an elastic arm and a locking catch; the locking catch protrudes from the elastic arm and is snap-engaged with the guide needle bracket; when the unlocking portion comes into contact with the elastic arm, the elastic arm is driven to undergo elastic deformation, thereby releasing the snap engagement between the locking catch and the guide needle bracket.

4. The applicator according to claim 3, wherein an end of the elastic arm is provided with a bent portion that bends toward an inner wall of the base, and the bent portion is configured to come into contact with the unlocking portion to release the snap engagement between the locking catch and the guide needle bracket .

5. The applicator according to claim 4, wherein the transmitter bracket further comprises a transmitter mounting portion, the transmitter is connected to the transmitter mounting portion, and the guide needle bracket is disposed on a side of the transmitter mounting portion facing the base; one end of the elastic arm is connected to the side of the transmitter mounting portion facing the base, and the other end of the elastic arm extends through the guide needle bracket; the locking catch is located on a side wall of the elastic arm facing the guide needle bracket, and the locking catch is snap-engaged with a top portion of the guide needle bracket; the bent portion is disposed at an end of the elastic arm facing away from the guide needle bracket.

6. The applicator according to claim 5, wherein a plurality of locking portions are arranged around the center of the transmitter mounting portion; a plurality of unlocking portions are provided on the contact member, and the plurality of unlocking portions are arranged in one-to-one correspondence with the plurality of locking portions.

7. The applicator according to claim 5, wherein when the unlocking portion comes into contact with the bent portion, the end of the elastic arm facing away from the transmitter mounting portion is driven to contract toward the center of the transmitter mounting portion, thereby buffering the movement of the guide needle bracket away from the transmitter mounting portion.

8. The applicator according to any one of claims 1 to 7, wherein the base is provided with an accommodating cavity, the accommodating cavity is provided with a first opening on a side facing the human body, and the contact member is movably disposed in the accommodating cavity; when the base moves toward or away from the contact side, the contact member is retracted into or extended out of the accommodating cavity from the first opening; when the base moves toward the contact side, the guide needle and part of the transmitter are driven to be inserted into the human body, and then the locking of the guide needle bracket by the locking portion is released.

9. The applicator according to claim 8, wherein a first limiting groove is provided on an inner wall of the accommodating cavity, and the first limiting groove extends in a direction perpendicular to the first opening; the contact member comprises a contact body and a limiting portion, the limiting portion is connected to the contact body and movably disposed in the first limiting groove to guide the contact body to move along an extending direction of the first limiting groove.

10. The applicator according to claim 8, wherein a resistance rib is provided on an inner wall of the accommodating cavity; the contact member comprises a contact body and a resistance arm, the contact body is movably connected to the inner wall of the accommodating cavity, one end of the resistance arm is connected to the contact body, and the other end of the resistance arm is provided with a resistance arm catch which is configured to cooperate with the resistance rib.

11. The applicator according to claim 8, wherein a first positioning arm is provided on the inner wall of the accommodating cavity, and the first positioning arm is provided with a positioning groove; the transmitter bracket comprises a transmitter mounting portion and a second positioning arm, one end of the second positioning arm is connected to the transmitter mounting portion, the other end of the second positioning arm is provided with a positioning arm catch, and the positioning arm catch is snap-engaged with the positioning groove.

12. The applicator according to claim 8, further comprising a cover, wherein the cover is detachably connected to the base to close the first opening.

13. The applicator according to claim 8, wherein the transmitter bracket comprises a transmitter mounting portion, a side of the transmitter mounting portion facing the first opening is provided with a mounting groove, the transmitter is mounted in the mounting groove, the guide needle extends out from a middle portion of the transmitter, and an analyte sensor of the transmitter is disposed in the guide needle.

14. The applicator according to claim 13, wherein the guide needle bracket is disposed on a side of the transmitter mounting portion facing away from the first opening; the guide needle bracket is provided with a receiving cavity, and a second opening is formed on a side of the receiving cavity facing the transmitter mounting portion; the elastic member is disposed in the receiving cavity, one end of the elastic member abuts against an inner wall of the guide needle bracket, and the other end thereof abuts against the transmitter mounting portion.

15. The applicator according to claim 14, wherein a middle portion of the guide needle bracket is provided with a connecting arm, the connecting arm is provided with a connecting catch, a rear end of the guide needle is provided with a connecting groove, and the connecting groove is snap-engaged with the connecting catch; the transmitter mounting portion is provided with a connecting through hole opposite to the connecting arm, and the guide needle passes through the transmitter mounting portion via the connecting through hole.

16. The applicator according to claim 14, wherein a side of the guide needle bracket facing away from the second opening is provided with a plurality of first clearance holes; the locking portion comprises an elastic arm, and the first clearance holes are configured to allow the elastic arm to pass through.

17. The applicator according to claim 14, wherein a second limiting groove is provided on an inner side wall of the guide needle bracket, and an extending direction of the second limiting groove is perpendicular to the second opening; the locking portion includes an elastic arm, and the second limiting groove is configured to cooperate with the elastic arm to guide the guide needle bracket to move in a direction perpendicular to the second opening.

18. The applicator according to claim 13, wherein a mounting arm is provided on a side wall of the mounting groove, a mounting catch is provided on a side of the mounting arm facing the transmitter, the transmitter is provided with an engaging slot corresponding to the mounting catch, and the mounting catch is snap-engaged with the engaging slot.

19. The applicator according to claim 13, wherein the contact member is provided with an inner cavity, and a side of the inner cavity facing the human body is provided with a third opening; the transmitter mounting portion and the guide needle bracket are located in the inner cavity; a side of the contact member facing away from the third opening is provided with a through hole, the guide needle bracket is arranged in the through hole, and the through hole is configured to allow the guide needle bracket to move away from the transmitter mounting portion.

20. The applicator according to claim 19, wherein a plurality of second clearance holes are disposed on a side of the contact member away from the third opening; the base comprises a first positioning arm, and the second clearance holes are configured to allow the first positioning arm to pass through.

21. The applicator according to claim 19, wherein an inner wall of the contact member is further provided with a limiting structure, and the limiting structure is configured to cooperate with the transmitter bracket or the guide needle bracket.
